(19) Europäisches Patentamt
European Patent Office
Office européen des brevets

(11) EP 3 548 626 B1

# (12) EUROPÄISCHE PATENTSCHRIFT

(45) Veröffentlichungstag und Bekanntmachung des Hinweises auf die Patenterteilung:
**05.08.2020 Patentblatt 2020/32**

(21) Anmeldenummer: **16816216.2**

(22) Anmeldetag: **30.11.2016**

(51) Int Cl.:
***C12P 5/02*** (2006.01)

(86) Internationale Anmeldenummer:
**PCT/EP2016/079215**

(87) Internationale Veröffentlichungsnummer:
**WO 2018/099547 (07.06.2018 Gazette 2018/23)**

(54) **VERFAHREN ZUR ERZEUGUNG VON BIOGAS AUS FASERHALTIGEM SUBSTRAT**

PROCESS FOR PRODUCING BIOGAS FROM FIBROUS SUBSTRATE

PROCÉDÉ DE PRODUCTION DE BIOGAZ À PARTIR D'UN SUBSTRAT FIBREUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**09.10.2019 Patentblatt 2019/41**

(73) Patentinhaber: **Verbio Vereinigte Bioenergie AG 04109 Leipzig (DE)**

(72) Erfinder:
• **LÜDTKE, Oliver 04416 Markkleeberg (DE)**
• **SCHLIMBACH, Michael 06120 Halle/Saale (DE)**

(74) Vertreter: **Gulde & Partner Patent- und Rechtsanwaltskanzlei mbB Wallstraße 58/59 10179 Berlin (DE)**

(56) Entgegenhaltungen:
WO-A1-2010/094115      WO-A1-2013/155631
WO-A1-2015/092003      US-A1- 2006 175 252

• **YI JING ET AL: "Effect of Increasing Total Solids Contents on Anaerobic Digestion of Food Waste under Mesophilic Conditions: Performance and Microbial Characteristics Analysis", PLOS ONE, Bd. 9, Nr. 7, Juli 2014 (2014-07), XP002767355,**
• **DHV Consultants BV & DELFT HYDRAULICS: "Training module # WQ - 10 How to measure dissolved, suspended& total solids", , 2. November 2002 (2002-11-02), XP002767606, Gefunden im Internet: URL:https://www.researchgate.net/file.Post FileLoader.html?id=574c67c840485482650e06f 2&assetKey=AS%3A367478443134976%401464 6250 96271 [gefunden am 2017-02-23]**

EP 3 548 626 B1

## Beschreibung

Die Erfindung betrifft ein Verfahren zur Erzeugung von Biogas aus faserhaltigem Substrat durch anaerobe Fermentation.

## Beschreibung des Standes der Technik

Der Abbau von organischem Substrat durch mikrobielle anaerobe Prozesse, auch Fermentationen genannt, ist bekannt. Bei der Erzeugung von Biogas, einem Gemisch überwiegend aus Methan und Kohlendioxid, erfolgt ein Abbau des organischen Substrats in mehreren Stufen (Hydrolyse, Versäuerung und Methanbildung). Bei dem eingesetzten Substrat handelt es sich um eine organische Substanz bzw. ein Gemisch verschiedener organischer Substanzen. Je nach Substrat und gewünschtem Produkt sind bestimmte Mikroorganismen oder Gruppen von Mikroorganismen für den Fermentationsprozess geeignet. Für die erfolgreiche Durchführung einer Fermentation sind neben der Substratversorgung auch die Versorgung mit Nährstoffen sowie die Einhaltung günstiger Prozessparameter wie zum Beispiel Temperatur und Druck sicherzustellen. Das organische Substrat wird in Abhängigkeit der Zusammensetzung unterschiedlich gut anaerob abgebaut. Sehr gut abbaubar sind einfache Moleküle sowie Kohlenhydrate, Proteine und Lipide. Schwerer bis gar nicht anaerob abbaubar sind die Makromoleküle der Faserbestandteile wie Hemicellulose und Cellulose sowie Lignin. Die nicht abbaubaren Faserbestandteile werden wieder aus dem Prozess ausgeschleust.

Die Biogasproduktion hat in den letzten Jahren zunehmend an Bedeutung gewonnen. Während zunächst die anaerobe Behandlung von Klärschlamm primär mit dem Ziel einer Schlammreduktion unter Bildung von Biogas im Zentrum des Interesses stand, lag der Fokus in jüngerer Vergangenheit auf der Gewinnung von Biogas aus landwirtschaftlicher Anbaubiomasse wie zum Beispiel Ganzpflanzensilage, teilweise auch kombiniert mit der Vergärung von Gülle oder anderen Exkrementen der Tierhaltung. Zunehmend in den Fokus rückt die Erzeugung von Biogas aus landwirtschaftlichen Reststoffen, da für diese keine Nahrungs- oder Futtermittelkonkurrenz besteht. Allerdings handelt es sich bei den landwirtschaftlichen Reststoffen in der Regel um faserhaltige und damit schwerer abbaubare Substrate. Für eine hohe Rentabilität ist eine möglichst hohe Biogasausbeute bezogen auf das eingesetzte faserhaltige organische Substrat erforderlich. Es gibt eine Reihe von technischen Vorschlägen, welche dieses Ziel verfolgen.

Stand der Technik zur Erreichung hoher Biogasausbeuten sind eine moderate, gleichbleibende Substratbelastung und eine ausreichende Versorgung mit Makro- und Mikronährstoffen sowie die Realisierung einer möglichst langen Verweilzeit des Substrats im Fermentationsprozess. Dabei ist im Hinblick auf die Wirtschaftlichkeit einer Anlage ein Kompromiss zwischen hoher Raumbelastung und langer Verweilzeit und damit verbundener hoher Biogasausbeute zu finden. Üblicherweise besteht der Kompromiss darin, die Anlagenleistung soweit zu steigern, bis die Prozessgüte messbar sinkt oder die Prozessstabilität abnimmt.

Bei der Vergärung von faserhaltigem Substrat ist der limitierende Faktor in der Regel die Durchmischbarkeit bzw. die reale Viskosität im Fermenter. Durch den hohen Fasergehalt ist die Hydrolyse der limitierende Abbauprozess.

Dem Fachmann ist bekannt, dass ab bestimmten Trockensubstanzgehalten (TS) die Viskosität des Fermenterinhalts stark zunimmt. Dadurch wird die Durchmischung und somit der konvektive Stofftransport und im Endeffekt die Biogasbildung erheblich beeinträchtigt. Für die Nassvergärung werden in der Fachliteratur sehr unterschiedliche Werte für maximal mögliche TS-Gehalte angegeben. So wird zum Beispiel im "Leitfaden Biogas" (ISBN 3-00-014333-5, 6. Auflage, 2013) für die Nassvergärung eine typische Grenze von 12% angegeben.

Ferner wird im "Leitfaden Biogas" beschrieben, dass es für die unterschiedlichen Substrate keine genau definierten Grenzen gibt:

"Bei Nassvergärungsverfahren sind in der Fermenterflüssigkeit Trockensubstanzgehalte von bis zu 12 Masseprozent vorzufinden. Als Faustregel gilt eine Grenze von 15 Masseprozent für die Pumpbarkeit des Mediums, jedoch ist diese Angabe qualitativ und nicht für alle Einsatzstoffe zu werten. Einige Substrate mit feindisperser Partikelverteilung und hohen Gehalten an gelösten Stoffen sind auch bei TS-Gehalten von bis zu 20 Masseprozent noch pumpfähig, beispielsweise dispergierte Speisereste aus dem Tankfahrzeug. Hingegen liegen andere Substrate bereits bei 10 bis 12 Masseprozent in der stapelbaren Form vor, wie z.B. Obst- und Gemüseschalen."

US 2006/175252 A1 offenbart ein Verfahren zur Erzeugung von Biogas aus Abwasser als Substrat. Durch anaerobe Fermentation in einem zwei-Phasen-System wird das Substrat in Abhängigkeit von mehreren Faktoren, z.B. gewünschte hydraulische Verweilzeit, organische Beladungsrate und erwartete Absinkcharakteristika des Schlamms, einem Fermenter zugeführt. In diesem wird das Substrat unter Rühren einer Nassvergärung zur Erzeugung von Biogas unterzogen. Der fermentiertes Substrat enthaltende Ablauf wird aus dem Fermenter abgezogen und die TSS-Gehalte (total suspended solids) im Fermenter und im Ablauf werden ermittelt.

Der Erfindung liegt insbesondere das Problem zugrunde, ein Verfahrenskonzept zur effizienten und kostengünstigen Erzeugung von Biogas aus beliebigem faserhaltigem Substrat bereitzustellen.

**Zusammenfassung der Erfindung**

Das Problem wird durch ein Verfahren zur Erzeugung von Biogas aus faserhaltigem Substrat durch anaerobe Fermentation nach Anspruch 1 gelöst oder zumindest gemindert, welches folgende Schritte umfasst:

a) Zuführung eines faserhaltige Substrats zusammen mit Prozessflüssigkeit in einen anaerobe Mikroorganismen enthaltenden Fermenter in Abhängigkeit des Gehalts der suspendierten Trockensubstanz (TSS-Gehalts) in diesem Fermenter,
b) Erzeugen von Biogas aus dem faserhaltigen Substrat in diesem Fermenter durch Nassvergärung,
c) Abziehen des fermentiertes faserhaltiges Substrat enthaltenden Ablaufs aus dem Fermenter und Bestimmung des TSS-Gehalts,
d) Vergleich des ermittelten TSS-Gehalts mit dem festgelegten Zielbereich,
e) Wiederholen von Schritt (a) mit in Abhängigkeit des Ergebnisses von Schritt (d) angepassten Mengen, um den Zielbereich des TSS im Fermenter einzuhalten.

Unter faserhaltigem Substrat werden in dieser Anmeldung Substrate ohne signifikante Mengen freien Wassers und mit einem signifikanten Gehalt an Faserbestandteilen wie zum Beispiel Lignocellulosefasern verstanden. Dies beinhaltet zum Beispiel aber nicht ausschließlich Heu oder Gräser aus der Landschaftspflege, Stroh oder andere Rückstände der Getreideproduktion, Rapsstroh, Erbsenstroh, Miscanthus, Rohrglanzgras, Hirse oder andere Energieganzpflanzen oder Pflanzenteile bzw. faserhaltige industrielle Abfälle zum Beispiel aus der holzverarbeitenden Industrie.

Biogas im Sinne dieser Anmeldung umfasst jegliches im Rahmen einer einstufigen und/oder mehrstufigen Vergärung mikrobiell gebildete Gas. Biogas enthält vor allem $CO_2$, Methan und/oder Wasserstoff und Wasser sowie je nach Substrat und Gärprozess Ammoniak und Schwefelwasserstoff.

Die suspendierte Trockensubstanz (TSS) beschreibt einen partikulären Anteil der Trockensubstanz (TS). Bei faserhaltigen Medien wird die TSS maßgeblich vom Fasergehalt des Mediums bestimmt. Die TSS ergibt zusammen mit der gelösten Trockensubstanz die gesamte Trockensubstanz. Je nach Analysenmethode werden sehr kleine Partikel unterhalb der Trennkorngröße der Analysenmethode der gelösten Trockensubstanz zugeordnet.

In einer bevorzugten Ausführungsform ist das erfindungsgemäße Verfahren so ausgestaltet, dass es sich bei dem TSS-Gehalt um den Faser-Gehalt handelt.

In einer weiteren bevorzugten Ausführungsform ist das erfindungsgemäße Verfahren so ausgestaltet, dass die Zufuhr von faserhaltigem Substrat zusammen mit Prozessflüssigkeit so erfolgt, dass ein TSS-Gehalt im Gärmedium zwischen 4% bis 10%, vorzugsweise zwischen 5% bis 8%, besonders bevorzugt zwischen 6 bis 7% eingestellt wird.

In einer weiteren bevorzugten Ausführungsform ist das erfindungsgemäße Verfahren so ausgestaltet, dass die Zufuhr von faserhaltigem Substrat zusammen mit Prozessflüssigkeit so erfolgt, dass ein Faser-Gehalt im Gärmedium zwischen 4% bis 10%, vorzugsweise zwischen 5% bis 8%, besonders bevorzugt zwischen 6 bis 7% eingestellt wird.

In einer weiteren bevorzugten Ausführungsform ist das erfindungsgemäße Verfahren so ausgestaltet, dass dem Fermentationsprozess Nährstoffe und Spurenelemente direkt oder indirekt zugeführt werden.

In einem weiteren Aspekt ist das Verfahren so ausgestaltet, dass der Fermenterinhalt während der Fermentation gerührt wird.

In einer weiteren bevorzugten Ausführungsform ist das erfindungsgemäße Verfahren so ausgestaltet, dass der Ablauf einer Fest-Flüssig-Trennung unterzogen wird und dabei feuchter faserhaltiger Gärrest und eine Prozessflüssigkeit erzeugt werden.

In einer weiteren bevorzugten Ausführungsform ist das erfindungsgemäße Verfahren so ausgestaltet, dass die in einer Fest-Flüssig-Trennung erzeugte Prozessflüssigkeit wieder einem Fermenter zugeführt wird.

In einem weiteren Aspekt ist das Verfahren so ausgestaltet, dass Frischwasser einem Fermenter zugeführt wird.

In einem weiteren Aspekt ist das Verfahren so ausgestaltet, dass als Prozessflüssigkeit pumpfähiges vergärbares Substrat eingesetzt wird.

In einem weiteren Aspekt ist das Verfahren so ausgestaltet, dass das faserhaltige Substrat vor der Vergärung vermahlen wird.

In einer weiteren bevorzugten Ausführungsform ist das erfindungsgemäße Verfahren so ausgestaltet, dass der TSS-Gehalt eines Biogas-Ablaufs nach folgender Methode bestimmt wird, indem:

a) Aliquote einer Ablauf-Probe für verschiedene Analysen bereitgestellt werden,
b) aus einem Aliquot der Gehalt der Trockensubstanz (TS-Gehalt) dieser Probe gemessen wird,
c) ein anderes Aliquot dieser Probe zunächst zentrifugiert und feinfiltriert wird und aus dem Filtrat der Gehalt der gelösten Trockensubstanz (TSgel.-Gehalt) bestimmt wird, und
d) der TSS-Gehalt aus dem TS-Gehalt und dem TSgel.-Gehalt des Filtrats berechnet wird. Das erfindungsgemäße Verfahren betrifft insbesondere auch alle Kombinationen der oben beschriebenen, bevorzugten Ausführungsformen.

Die Erzeugung von Biogas aus faserhaltigem Substrat erfolgt besonders zweckmäßig und kostengünstig in einem einstufigen Verfahren. Dabei laufen alle Abbauschritte parallel in einem durchmischten Fermenter ab. Es sind auch mehrstufige Vergärungsverfahren möglich, bei denen zum Beispiel eine Hauptvergärung und Nachvergärung stattfindet oder mehrere Fermenter parallel betrieben werden.

Die Erzeugung von Biogas kann auch durch Entkopplung der Abbauschritte in einen Hydrolyseprozess und einen Vergärungsprozess erfolgen. Im Hydrolysereaktor findet dann überwiegend die Hydrolyse und Versäuerung des Substrats statt. Im sich anschließenden Vergärungsprozess findet dann die Methanbildung statt, so dass das in diesem Reaktor gebildete Gas den überwiegenden Teil des aus dem Substrat gebildeten Methans enthält.

Eine sehr gute Durchmischung wird bei einer Hydrolyse bzw. Vergärung im Nassfermentationsverfahren erreicht, wenn das faserhaltige Substrat mit Prozessflüssigkeit zu einer Suspension angemischt wird.

Das faserhaltige Substrat wird gegebenenfalls nach einer Zerkleinerung zusammen mit Prozessflüssigkeit als Suspension einem anaeroben Fermentationsprozess zugeführt. Die Zerkleinerung erfolgt zweckmäßig auf Partikelgrößen im Bereich 1mm bis 20mm, bevorzugt im Bereich 2mm bis 15mm, besonders bevorzugt im Bereich 3mm bis 10mm, ganz besonders bevorzugt im Bereich 4mm bis 6mm.

In einem anaerobe Mikroorganismen enthaltenden Fermenter findet vorzugsweise unter ständigem Rühren zur Verbesserung des Stoffaustauschs der anaerobe Abbau der organischen Substanz zu Biogas statt. Falls durch die zugeführten Substrate keine ausreichende Versorgung mit Nährstoffen und Spurenelementen erfolgt, werden diese dem Gärprozess in geeigneter Weise zugeführt.

Während ein Teil der TS zu Biogas abgebaut wird, wird ein anderer Teil in lösliche Bestandteile abgebaut, ein weiterer Teil des Substrats (z.B. Faserbestandteile) unterliegt keinem oder nur einem geringen Abbau und verbleibt als suspendierte TS im Gärmedium. Insbesondere Ligninverbindungen werden anaerob fast gar nicht abgebaut.

Um die Biogasbildung möglichst effizient zu gestalten ist es notwendig möglichst hohe Substratkonzentrationen im Fermenter zu erreichen. Dadurch wird die Verweilzeit im Fermenter maximiert und eine hohe Raumausbeute bezogen auf das Fermentervolumen erreicht. Gleichzeitig darf die Substratkonzentration nicht so weit gesteigert werden, dass bedingt durch einen hohen TS-Gehalt die Viskosität des Gärmediums derart ansteigt, dass eine ausreichende Durchmischung nicht mehr gewährleistet ist.

Die Viskosität eines partikulären Gärmediums ist nur schlecht messbar und eine Übertragung des Messergebnisses auf die Güte der Durchmischung kaum möglich. Auch der TS-Gehalt kann die Durchmischbarkeit nur unzureichend widerspiegeln, da er sich aus verschiedenen Fraktionen (z.B. gelöste und suspendierte TS) zusammensetzt, welche sich unterschiedlich stark auf die Durchmischungseigenschaften des Gärmediums auswirken.

Es wurde gefunden, dass als sehr gut geeignete Messgröße die suspendierte TS (TSS) zur Regelung der Substratzufuhr im Biogasprozess geeignet ist. Während die gelöste TS eine untergeordnete Rolle für die Durchmischbarkeit spielt, ist der Einfluss der TSS dominant. Überraschenderweise wurde gefunden, dass für die Vergärung von faserhaltigem Substrat der Gehalt der gelösten TS keinen Einfluss auf die Ausbeute hat.

Die TSS kann durch geeignete Methoden im Gärmedium bestimmt werden und ist damit für den gesamten Fermenterinhalt bekannt. Damit ist es möglich auch bei unterschiedlicher Substratzusammensetzung die Auslastung des Fermenters durch eine Anpassung der Substratzufuhr und/oder Zufuhr der Prozessflüssigkeit immer im optimalen Bereich zu fahren. Dabei ist es von Vorteil, aber nicht zwingend erforderlich, wenn auch die TS-Zusammensetzung der Substrate und der Prozessflüssigkeit bekannt sind.

In einigen Anwendungen kann es hilfreich sein, anstelle des TSS den Fasergehalt des Gärmediums als Regelungsgröße zu verwenden. Der Fasergehalt lässt sich durch unterschiedliche Methoden zum Beispiel als Rohfasergehalt nach der Weender Analyse bestimmen. Möglich ist auch die Verwendung der ADF- bzw. ADL-Werte der erweiterten Weender Analyse als Fasergehalt.

Die Prozessflüssigkeit kann aus prozessintern gewonnener Flüssigkeit und/oder Wasser bestehen. Durch eine Fest-Flüssig-Trennung können aus dem Gärmedium insbesondere partikuläre Bestandteile oberhalb der dem jeweiligen Trennprozess eigenen Trennkorngröße abgetrennt werden. Die dabei entstehende Prozessflüssigkeit kann wieder zum Anmischen von neuem Substrat verwendet bzw. in den Fermenter zurückgeführt werden.

In Versuchen wurde überraschenderweise gefunden, dass für ein bestimmtes Fermentationssystem bestehend aus einem Fermenter und der zugehörigen Rührtechnik ein substratunabhängiger Grenzwert für die suspendierte Trockensubstanz im Gärmedium existiert bei dem die Biogasbildung optimal ist. Oberhalb dieses Grenzwertes kann es durch mangelnde Durchmischung zu einer lokalen Überlastung der Biologie und damit zu einer Säureakkumulation im Gärmedium kommen. Ein weiterer Effekt ist die Verringerung der Gasausbeute.

Es wurde gefunden, dass die Biogasbildung optimal ist, wenn ein TSS-Gehalt im Gärmedium zwischen 4% bis 10%, vorzugsweise zwischen 5% bis 8%, besonders bevorzugt zwischen 6 bis 7% eingestellt wird.

Das Regelungskonzept ist ebenso für eine mehrstufige Fahrweise zum Beispiel mit Hauptvergärung und Nachvergärung oder bei parallel betriebenen Fermentationen anwendbar. In diesen Fällen kann eine Regelung für jeden einzelnen Fermenter erfolgen. Ebenso kann das Regelungskonzept bei mehrstufigen Prozessen mit Hydrolyse und nachgeschalteter Hauptvergärung Anwendung finden.

Weitere vorteilhafte Weiterbildungen der Erfindung sind den abhängigen Ansprüchen oder der nachfolgenden Beschreibung zu entnehmen.

Kurzbeschreibung der Figuren

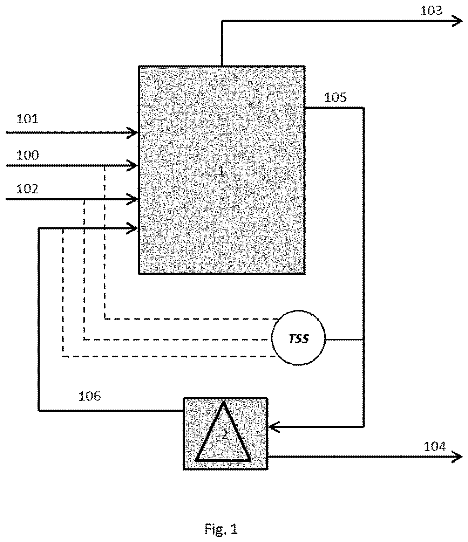

Fig. 1    zeigt ein Fließschema des Versuchsaufbaus von Beispiel 3

**Detaillierte** Beschreibung der Erfindung

Beispiel 1

Die Bestimmung der TSS wird nachfolgend detailliert beschrieben.

Prinzip: Von einer Probe werden die Trockensubstanz und die gelöste Trockensubstanz durch Verdampfen des Wassers einer unverdünnten Probe bzw. eines verdünnten Filtrates im Muffelofen bestimmt. Mit diesen Werten kann die suspendierte Trockensubstanz (TSS) berechnet werden.

Material: 50 ml Zentrifugenröhrchen, Einmalspritze 5 ml, Spritzenvorfilter 0,2 $\mu$m, Analysenwaage, Zentrifuge, Muffelofen Nabertherm B180, feuerfeste Glasschale, Exsikkator, Tiegelzange, destilliertes Wasser.

Durchführung: Die Bestimmungen der Trockensubstanz und der gelösten Trockensubstanz sind als Doppelbestimmungen durchzuführen. Die zu untersuchende Probe wird vor der Entnahme durch Schütteln homogenisiert. Für die TS-Bestimmung wird je eine Schale gewogen und das Gewicht (m1) notiert. 10,00 g $\pm$1,0 g der Probe werden in die Schale gefüllt und das Endgewicht wird ebenfalls notiert (m2). Für die Bestimmung der gelösten Trockensubstanz werden in einem 50 ml Zentrifugenröhrchen 10,00 g $\pm$ 1,0 g Probe eingewogen und die exakte Masse notiert (m4). Man füllt auf 50,00 g $\pm$1,0 g mit dest. Wasser auf und notiert die Gesamtmasse (m5). Der Inhalt des Röhrchens wird durch Schütteln homogenisiert und bei 4 400 u für 5 min zentrifugiert. Man wiegt eine Schale und notiert das Gewicht (m6).Vom Zentrifugat werden 10,00 g $\pm$ 1,0 g über einen 0,2 $\mu$m Vorspritzenfilter in die Schale gegeben. Man notiert das Endgewicht (m7). Mit Hilfe einer Tiegelzange werden alle Schalen im Muffelofen platziert um das Wasser zu verdampfen. Man heizt mit 5 °C/min auf 105 °C hoch und nach Erreichen der Temperatur wird diese für 3 Stunden gehalten. Danach werden die Proben zum Abkühlen für ca. 20-30 min in den Exsikkator gestellt. Nach dem Abkühlen werden die Schalen ausgewogen und die Masse notiert (Masse des Probe der TS Bestimmung m3, Masse des Probe für die gelöste TS-Bestimmung m8). Die Berechnung der Trockensubstanz TS und der gelösten Trockensubstanz TSgel. erfolgt nach den unten stehenden Formeln. Aus der Differenz zwischen dem Wert für die Trockensubstanz und dem Wert für die gelöste Trockensubstanz kann die suspendierte Trockensubstanz TSS berechnet werden. Ergibt die Auswertung einen Gehalt von weniger als 1% für den verdünnten gelösten TS-Gehalt TS*gel. so ist die Bestimmung mit einer kleineren Verdünnung zu wiederholen.

Berechnung:

Verdünnungsfaktor VF für die Probeneinwaage:

$$VF = m5 / m4$$

m4    Masse der Probe
m5    Gesamtmasse nach Verdünnung

Der Gehalt an Trockensubstanz der Ausgangsprobe berechnet sich nach:

$$TS = (m3 - m1) / (m2 - m1)$$

m1    Leermasse des Tiegels der TS-Bestimmung
m2    Masse des vollen Tiegels der TS-Bestimmung vor 105 °C
m3    Masse des Tiegels der TS-Bestimmung nach 105 °C

Die gelöste Trockensubstanz der verdünnten Probe TS*gel. berechnet sich nach:

$$TS^*gel. = (m8 - m6) / (m7 - m6)$$

m6    Leermasse des Tiegels der TSgel.-Bestimmung
m7    Masse des vollen Tiegels der TSgel.-Bestimmung vor 105 °C
m8    Masse des Tiegels der TSgel.-Bestimmung nach 105 °C

Der um die suspendierte Trockensubstanz TSS korrigierte Wert für die gelöste Trockensubstanz TSgel. kann nach folgender Gleichung ermittelt werden:

$$TSgel. = VF * (TS^*gel. * (1 - TS / VF )) / (1 - TS^*gel.)$$

TS*gel.    bestimmte gelöste Trockensubstanz in der verdünnten Probe
TS         Trockensubstanz der Probe

Die Differenz zwischen Trockensubstanz TS und gelöster Trockensubstanz TSgel. ist die suspendierten Trockensubstanz TSS:

$$TSS = TS - TSgel.$$

Beispiel 2

Eine weitere Methode zur Bestimmung des TSS-Gehalts wird nachfolgend detailliert beschrieben.
Die Bestimmung der TSS erfolgt durch Vakuumfiltration einer definierten Probenmenge durch einen Cellulose-Acetatfilter mit einer Porengröße von 0,45 $\mu$m.
Der 0,45 $\mu$m Cellulose-Acetatfilter kann zur Entfernung der wasserlöslichen Verunreinigungen mit 150 ml Wasser vorgewaschen werden. Der Filter wird anschließend bei 105 °C für mindestens eine Stunde bis zur Massekonstanz getrocknet und bis zur Verwendung im Exsikkator gelagert. Es ist darauf zu achten, dass der Filter nicht durch Staub verunreinigt wird.
Der Filter wird vor der Filtration gewogen (m1) und anschließend in den Trichter einer Filtriereinheit gelegt.
Die zu untersuchende Probe wird durch kräftiges Schütteln homogenisiert und ohne Standzeit werden, von dieser Probe ca. 10 g (m2) in einen 25 ml Messzylinder gefüllt. Die Probe wird mittels Vakuum filtriert und der Messzylinder zweimal mit 25 ml Wasser gespült. Anschließend werden der Filter und der Trichter mit weiteren 50 ml Wasser gespült und der Filter trocken gesaugt. Die abschließende Trocknung des Filters erfolgt bei 105 °C im Trockenschrank bis zur Massekonstanz (m3). Aus dem Endgewicht kann über die folgende Gleichung die TSS berechnet werden.

m1    Masse des unbeladenen Filters

m2    Probeneinwaage

m3    Masse des beladenen Filters nach Filtration

$$TSS = (m3 - m1) / m2$$

Beispiel 3

Die Möglichkeit einer technischen Ausführung des Verfahrens sei im Folgenden anhand der Vergärung von Stroh erläutert. Stroh steht dabei nur beispielhaft für die in dieser Anmeldung betrachteten faserhaltigen Substrate. Eine Übertragung des Sachverhalts auf die Anwendung anderer geeigneter Substrate ist dem Fachmann ohne weiteres möglich.
Das Beispiel nimmt Bezug auf Fig. 1. Darin bedeuten:

100    Stroh

101    Nährstoffe/Spurenelemente

102    Wasser

103    Biogas

104    Reststoff

105    Ablauf

106    Prozessflüssigkeit

1    Fermentation

2    Fest-Flüssig-Trennung

Stroh ist als landwirtschaftlicher Reststoff sehr gut als Substrat für die Biogasgewinnung geeignet. Allerdings enthält Stroh signifikante Mengen schwer abbaubarer Substanzen, welche anaerob kaum bzw. nicht in wirtschaftlich vertretbaren Zeiträumen abgebaut werden. Je nach Strohsorte und Erntezeitpunkt sowie Lagerungsbedingungen kann die Substratzusammensetzung stark variieren. Rapsstroh fällt in Deutschland zum Beispiel mit einer Feuchte von etwa 30% an, während Weizenstroh bei trockener Witterung und sachgerechter Lagerung meist nur ca. 9% Feuchte enthält. Reisstroh enthält im Vergleich besonders viele Mineralstoffe und kann Rohaschegehalte von über 20% der TS enthalten.
Typische Bandbreiten einiger Inhaltsstoffe von Stroh sind nachfolgender Tabelle zu entnehmen:

|  | Min | Max |
| --- | --- | --- |
| Feuchte | 5% der OS | 40% der OS |
| TS | 60% der OS | 95% der OS |
| oTS | 75% der TS | 97% der TS |
| Rohasche | 3% der TS | 25% der TS |
| Lignin (ADL) | 3% der TS | 20% der TS |
| Stickstoff (Kjeldahl) | 0,3% der OS | 1,1% der OS |

Je nach Strohsorte und Qualität kann sich das Biogaspotenzial erheblich unterscheiden. Auch die sich im Fermentationsprozess nach Abbau der abbaubaren Substanz verbleibende Trockensubstanz kann sich deutlich unterscheiden. Bei Reisstroh zum Beispiel verbleibt durch den hohen Mineralstoffgehalt ein erheblicher Teil Trockensubstanz nach der Vergärung erhalten. Gleichzeitig wird aber auch ein signifikanter Teil der Mineralstoffe in gelöste Trockensubstanz umgewandelt. Dieser Anteil ist für das Durchmischungsverhalten des Gärmediums von nachrangiger Bedeutung.
In einer Versuchsanlage bestehend aus einem kontinuierlichen Rührkessel (Fermenter, 1) wurden Biogasversuche durchgeführt. Der schematische Aufbau ist Fig. 1 zu entnehmen. Nachdem das System mit Inoculum, Wasser und Weizenstroh als Substrat unter Zugabe von Nährstoffen und Spurenelementen angefahren wurde, erfolgte im weiteren Verlauf ein quasikontinuierlicher Betrieb bei konstantem Füllstand mit der gleichen Charge Weizenstroh. Täglich wurde dem System Ablauf (105) entnommen und frisches, vermahlenes Stroh (100) sowie Prozessflüssigkeit (106) und Wasser (102) zugeführt. Die Nährstoff- und Spurenelementzugabe (101) erfolgte in regelmäßigen Intervallen.
Ein kleiner Teil des Ablaufs (105) wurde analysiert. Der überwiegende Teil des Ablaufs (105) wurde einer Fest-Flüssig-Trennung in einem Dekanter (2) unterzogen. Dadurch wurden ein rieselfähiger faserhaltiger Gärrest (104) sowie eine Prozessflüssigkeit (106) gewonnen. Die gewonnene Prozessflüssigkeit (106) wurde zurück in den Fermenter (1) gefahren. Zusätzlich zur Prozessflüssigkeit (106) wurden noch 15% Frischwasser (102) eingesetzt.
Im stationären Zustand wurde ein TSS-Gehalt von 6,5% im Gärmedium realisiert. Der Gehalt an gelöster Trockensubstanz lag zu diesem Zeitpunkt bei 2,0%.
In weiteren Versuchen wurde durch erhöhte Substratzufuhr und anteiliger Verringerung der Wasserzufuhr der TS-Gehalt im Gärmedium erhöht. Bei TS-Gehalten über 8,5% insbesondere über 9% und einem damit verbundenem Anstieg des TSS-Gehalts auf Werte über ca. 6,8% kam es zu einer Akkumulation von Essigsäure im Gärmedium, welche auf eine sich signifikant verschlechterte Durchmischung zurückzuführen war. Über Schaugläser war feststellbar, dass bei derart hohem TSS-Gehalt nur noch eine lokale Durchmischung in unmittelbarer Nähe des Rührwerks erfolgte, während bei einem TSS-Gehalt von 6,5% im Gärmedium eine großvolumige Durchmischung zu beobachten war.
Durch einen Substratwechsel auf Reisstroh mit einem Rohaschegehalt von ca. 20% der TS (statt 7% der TS bei Weizenstroh) verblieb signifikant mehr nicht abbaubare TS im System. Zunächst wurde der Fermentationsprozess so gefahren, dass ein gleichbleibender TS-Gehalt von etwa 8,5% im Gärmedium eingestellt wurde. Dies war nur durch eine im Vergleich zu Weizenstroh geringere Substratzufuhr möglich.

Durch Messwerte im Ablauf wurde festgestellt, dass sich der TSS-Gehalt im Gärmedium nach dem Substratwechsel stetig verringerte während der Gehalt an gelöster TS anstieg. Daraufhin wurde die Zufuhr von Reisstroh angehoben und die Zufuhr von Prozessflüssigkeit so angepasst, dass wieder ein TSS von 6,5% im Gärmedium eingestellt wurde. Der Anteil gelöster TS erhöhte sich bei der Verwendung von Reisstroh entsprechend auf ca. 3,0% und damit der Gesamtgehalt der TS im Gärmedium auf ca. 9,5%. Ein stabiler Betrieb bei derart hohem TS war bei Weizenstroh nicht möglich.

Eine weitere Steigerung der Gehalte an TSS bzw. TS führte wie auch schon beim vorherigen Versuch mit Weizenstroh zu einer Akkumulation von Essigsäure in Folge einer sich verschlechternden Durchmischung.

In weiteren Versuchen wurde das Regelungskonzept auch für andere Substrate bestätigt.

**Patentansprüche**

1. Verfahren zur Erzeugung von Biogas aus faserhaltigem Substrat durch anaerobe Fermentation **gekennzeichnet dadurch, dass**

   a) das faserhaltige Substrat zusammen mit Prozessflüssigkeit einem anaerobe Mikroorganismen enthaltenden Fermenter in Abhängigkeit des ermittelten Gehalts der suspendierten Trockensubstanz (TSS-Gehalts) in diesem Fermenter zugeführt,
   b) das faserhaltige Substrat in diesem Fermenter einer Nassvergärung zur Erzeugung von Biogas unterzogen,
   c) der fermentiertes faserhaltiges Substrat enthaltende Ablauf aus dem Fermenter abgezogen und der TSS-Gehalt im Fermenter ermittelt,
   d) der ermittelte TSS-Gehalt mit einem festgelegten Zielbereich verglichen und
   e) in Abhängigkeit des Ergebnisses von d) Schritt a) mit angepassten Mengen wiederholt wird, um den Zielbereich des TSS im Fermenter einzuhalten.

2. Verfahren nach Anspruch 1, **dadurch gekennzeichnet, dass** es sich bei dem TSS-Gehalt um den Faser-Gehalt handelt.

3. Verfahren nach einem der vorangegangenen Ansprüche, **dadurch gekennzeichnet, dass** die Zufuhr von faserhaltigem Substrat zusammen mit Prozessflüssigkeit so erfolgt, dass ein TSS-Gehalt im Gärmedium zwischen 4% bis 10%, vorzugsweise zwischen 5% bis 8%, besonders bevorzugt zwischen 6 bis 7% eingestellt wird.

4. Verfahren nach einem der vorangegangenen Ansprüche, **dadurch gekennzeichnet, dass** die Zufuhr von faserhaltigem Substrat zusammen mit Prozessflüssigkeit so erfolgt, dass ein Faser-Gehalt im Gärmedium zwischen 4% bis 10%, vorzugsweise zwischen 5% bis 8%, besonders bevorzugt zwischen 6 bis 7% eingestellt wird.

5. Verfahren nach einem der vorangegangenen Ansprüche, **dadurch gekennzeichnet, dass** der Fermenterinhalt während der Fermentation gerührt wird.

6. Verfahren nach einem der vorangegangenen Ansprüche, **dadurch gekennzeichnet, dass** der Ablauf einer Fest-Flüssig-Trennung unterzogen wird und dabei feuchter faserhaltiger Gärrest und eine Prozessflüssigkeit erzeugt werden.

7. Verfahren nach einem der vorangegangenen Ansprüche, **dadurch gekennzeichnet, dass** die in einer Fest-Flüssig-Trennung erzeugte Prozessflüssigkeit wieder einem Fermenter zugeführt wird.

8. Verfahren nach einem der vorangegangenen Ansprüche, wobei der TSS-Gehalt eines Biogas-Ablaufs nach einer Methode ermittelt wird, wobei:

   a) Aliquote einer Ablauf-Probe für verschiedene Analysen bereitgestellt werden,
   b) aus einem Aliquot der Gehalt der Trockensubstanz (TS-Gehalt) dieser Probe gemessen wird,
   c) ein anderes Aliquot dieser Probe zunächst zentrifugiert und feinfiltriert wird und aus dem Filtrat der Gehalt der gelösten Trockensubstanz (TSgel.-Gehalt) bestimmt wird, und
   d) der TSS-Gehalt aus dem TS-Gehalt des Biogas-Ablaufs und dem TSgel.-Gehalt des Filtrats berechnet wird.

**Claims**

1.  A process for producing biogas from fibrous substrate by anaerobic fermentation, **characterized by**

    a) supplying the fibrous substrate together with process liquid to a fermenter containing anaerobic microorganisms as a function of the determined suspended dry matter content (TSS content) in this fermenter,
    b) subjecting the fibrous substrate to wet fermentation in this fermenter in order to produce biogas,
    c) withdrawing the outflow containing fermented fibrous substrate from the fermenter and determining the TSS content in the fermenter,
    d) the determined TSS content is compared with a fixed target range, and
    e) as a function of the result from d), repeating step a) with adjusted quantities in order to comply with the target range for the TSS in the fermenter.

2.  The process according to claim 1, **characterized in that** the TSS content is the fibre content.

3.  The process according to one of the preceding claims, **characterized in that** the fibrous substrate is supplied together with process liquid in a manner such that a TSS content in the fermentation medium is set at between 4% and 10%, preferably between 5% and 8%, particularly preferably between 6 and 7%.

4.  The process according to one of the preceding claims, **characterized in that** the fibrous substrate is supplied together with process liquid in a manner such that a fibre content in the fermentation medium is set at between 4% and 10%, preferably between 5% and 8%, particularly preferably between 6 and 7%.

5.  The process according to one of the preceding claims, **characterized in that** the fermenter content is stirred during the fermentation.

6.  The process according to one of the preceding claims, **characterized in that** the outflow undergoes a solid-liquid separation, and in so doing, moist fibrous fermentation residue and a process liquid are produced.

7.  The process according to one of the preceding claims, **characterized in that** the process liquid produced in a solid-liquid separation is recycled to a fermenter.

8.  The process according to one of the preceding claims, wherein the TSS content of a biogas outflow is determined in accordance with a method wherein:

    a) aliquots of an outflow sample are provided for different analyses,
    b) the dry matter content (TS content) of this sample is measured from an aliquot,
    c) another aliquot of this sample is initially centrifuged and fine-filtered, and the dissolved dry matter content (TDS content) is determined from the filtrate, and
    d) the TSS content is calculated from the TS content of the biogas outflow and from the TDS content of the filtrate.


**Revendications**

1.  Procédé, destiné à générer du biogaz à partir d'un substrat fibreux, par fermentation anaérobie, **caractérisé en ce que**

    a) on alimente le substrat fibreux conjointement avec un liquide de processus vers un fermentateur contenant des micro-organismes anaérobies, en fonction de la teneur en substance sèche en suspension (teneur en SSS) déterminée dans ledit fermentateur,
    b) on soumet le substrat fibreux dans ledit fermentateur à une fermentation humide pour générer du biogaz,
    c) on soutire du fermentateur l'écoulement contenant du substrat fibreux fermenté et on détermine la teneur en SSS dans le fermentateur,
    d) on compare la teneur en SSS déterminée avec une fourchette cible fixée,
    e) en fonction du résultat de d), on réitère l'étape a) avec des quantités adaptées, pour respecter la plage cible de la SSS dans le fermentateur.

2.  Procédé selon la revendication 1, **caractérisé en ce que** la teneur en SSS est la teneur en fibres.

3. Procédé selon l'une quelconque des revendications précédentes, **caractérisé en ce que** l'alimentation de substrat fibreux, conjointement avec du liquide de processus s'effectue de telle sorte qu'une teneur en SSS comprise entre 4 % et 10 %, de préférence entre 5 % et 8 %, de manière particulièrement préférentielle, entre 6 % et 7 % se règle dans le milieu de fermentation.

4. Procédé selon l'une quelconque des revendications précédentes, **caractérisé en ce que** l'alimentation de substrat fibreux, conjointement avec du liquide de processus s'effectue de telle sorte qu'une teneur en fibres comprise entre 4 % et 10 %, de préférence entre 5 % et 8 %, de manière particulièrement préférentielle, entre 6 % et 7 % se règle dans le milieu de fermentation.

5. Procédé selon l'une quelconque des revendications précédentes, **caractérisé en ce qu'**on brasse le contenu du fermentateur pendant la fermentation.

6. Procédé selon l'une quelconque des revendications précédentes, **caractérisé en ce qu'**on soumet l'écoulement à une séparation solide/liquide et **en ce qu'**à cet effet, un résidu de fermentation fibreux humide et un liquide de processus soient générés.

7. Procédé selon l'une quelconque des revendications précédentes, **caractérisé en ce qu'**on réalimente vers un fermentateur le liquide de processus généré par une séparation solide/liquide.

8. Procédé selon l'une quelconque des revendications précédentes, lors duquel on détermine la teneur en SSS d'un écoulement de biogaz selon une méthode consistant à :

    a) mettre à disposition pour différentes analyses des aliquotes d'un échantillon d'écoulement,
    b) mesurer à partir d'une aliquote la teneur en substance sèche (teneur SS) dudit échantillon,
    c) centrifuger dans un premier temps et soumettre à un filtrage fin une autre aliquote dudit échantillon et déterminer à partir du produit filtré la teneur en substance sèche dissoute (teneur en gel SS), et
    d) calculer la teneur SSS à partir de la teneur SS de l'écoulement de biogaz et de la teneur en gel SS du produit filtré.

Fig. 1

**IN DER BESCHREIBUNG AUFGEFÜHRTE DOKUMENTE**

**In der Beschreibung aufgeführte Patentdokumente**

- US 2006175252 A1

**In der Beschreibung aufgeführte Nicht-Patentliteratur**

- Leitfaden Biogas. 2013